# EUROPEAN PATENT SPECIFICATION

(11) **EP 0 621 789 B1**
(45) Date of publication and mention of the grant of the patent: **14.04.1999**
(21) Application number: 93902884.1
(22) Date of filing: 31.12.1992
(51) Int. Cl.: A61K 38/00, C07K 7/06

(54) **PHARMACEUTICAL PENTAPEPTIDE COMPOSITIONS AND METHODS OF USE THEREOF**
PHARMAZEUTISCHE PENTAPEPTID-ZUSAMMENSETZUNG UND IHRE VERWENDUNG
COMPOSITIONS PENTAPEPTIDIQUES PHARMACEUTIQUES ET LEURS PROCEDES D'UTILISATION

(30) Priority: 02.01.1992 US 816205
(43) Date of publication of application: 02.11.1994
(73) Proprietor: CYTRAN LTD., Hamilton HM11 (BM)
(72) Inventor: IVANOV, Vadim Tikhonovich, Moscow, 117296 (RU); MIKHALYOVA, Inessa Ivanovna, Moscow, 117437 (RU); VASKOVSKY, Boris Victorovich, Moscow, 113162 (RU); MIKHALTSOV, Alexander Nickolaevich, Leningrad, 190031 (RU); KHAVINSON, Vladimir Khatskelevich, St. Petersburg, 197198 (RU); MOROZOV, Vyacheslav Grigorievich, St. Petersburg, 197198 (RU)
(74) Representative: Beresford, Keith Denis Lewis
(86) International application number: US9211381
(87) International publication number: WO9312810

(56) References cited:
- WO-A-93/08816
- US-A- 4 395 404
- US-A- 4 753 927
- US-A- 5 081 108
- US-A- 5 093 321
- US-A- 5 100 663

## Description

The present invention is directed to pentapeptide pharmaceutical compositions and uses thereof, in particular, uses thereof for making a pharmaceutical preparation for treatment of immunodepressed states and of opportunistic infections in immunodepressed states associated with acquired immune deficiency syndrome.

### BACKGROUND OF THE INVENTION

The present invention is based in part on the discovery that certain pentapeptides exhibit a broad range of efficacy for prevention and treatment of opportunistic infections in immunodepressed states, and for therapeutically effective treatment of immunodeficient states, particularly AIDS. This is believed to be highly unexpected for such a relatively small compound to exhibit such a broad range of activity. Furthermore, we have not found any significant side effects from the use of the peptides according to the present invention. Due to its simple nature, the pentapeptides are relatively inexpensive to manufacture.

As used herein, the terms "immunomodulator" and "immunomodulating" encompass the activity of enhancing or restoring the subject's immune system, as evidenced by measurable blood parameters and/or the patient's improved ability to combat infection or disease, and the ability to heal tissue. Hence, immunomodulation encompasses improvement of the immune system due to an immunodeficient state (for example, caused by removal of the thymus), and/or an immunodepressed state (for example, caused by exposure to radiation). Furthermore, the present invention provides for modulation of the immune system by lowering blood parameters and other indicia of the immune state if these indicia are abnormally elevated. The present invention encompasses the therapeutic method of treating the immunodeficient, immunodepressed or elevated immune state per se, thus providing prophylaxis against infection and disease, as well as a treatment of infection or disease indirectly by enhancing the immune system.

It is therefore an object of the present invention to provide pharmaceutical compositions of the pentapeptide which have broad immunomodulating activity, as well as activity for other uses such as treatment of infections, enhancement of metabolic processes, and many other uses.

These and other objects will be apparent from the following description and appended claims.

### DESCRIPTION OF THE PREFERRED EMBODIMENTS

The present invention provides pharmaceutical preparations comprising a pentapeptide of the Formula I, using the normal convention wherein the first named amino acid is the amino terminus and the last named amino acid is the carboxyl terminus.

Thr-Ala-Glx-Glx-Lys (I)

wherein Glx is Glu, Gln and all amino acids are in the L-configuration.

The compositions according to the present invention may be formulated into any convenient formulation which allows for the active ingredient to be absorbed into the blood stream. Intramuscular intravenous and intranasal forms of application are preferred. The preferred dosage rate of the active ingredient for intramuscular administration is about 50 to 100µg per dose for adults (for a 300 to 1000µg total treatment therapy); for infants up to 1 year old about 10µg per dose, for infants 1 to 3 years old about 10 to 20µg per dose; for infants 4 to 6 years old about 20 to 30µg per dose, for children 7 to 14 years old about 50µg per dose. All of the foregoing dosages are useful for a treatment of 3 to 10 days, depending upon the immunodeficiency level. The treatment may be repeated as needed, usually within 1 to 6 months.

For prophylactic uses against opportunistic infections in immunodeficient or immunodepressed patients, the intramuscular and/or intranasal single daily dose for adults may be from about 50 to 10µg, and for children about 10 to 50 µg per dose for treatment over 3 to 5 days.

For treatment of external infections, the peptide may be applied in single daily dosages of about 10µg (over 4 to 10 days) or as installations into the site of infection at about 5µg twice daily over about 4 to 5 days.

The peptide may be utilized intramuscularly as an injection solution with the active ingredient in a therapeutically effective immunopotentiating amount of about .001 to .01% by weight. If presented in the form of a tablet, capsule or suppository it is preferred that the active ingredient be present in an amount of about 0.1mg per tablet, suppository or capsule. If presented in such form, the capsule, suppository or tablet may also contain other conventional excipients and vehicles such as fillers, starch, glucose, etc.

The peptide may be obtained by conventional peptide synthesis, including the Merrifield solid state peptide synthesis technique. Typically an amino and side chain protected derivative of an activated ester of Glx is reacted with side-group protected L-Lys, attached to the solid phase as its C-terminus. After elimination of the alpha-amino protecting group, the next amino acid is added, etc. Cleavage of the peptide, removal of protection groups use purification, lyophilization, gel purification, and the like results in the desired product.

The purified peptide Thr-Ala-Glu-Glu-Lys, is a white powder (if lyophilized; otherwise, it is crystalline; hygroscopic and stable at less than 0°C in lyophilized form), as its TFA salt, it is soluble in water, ethanol; R_{f}=0.44 (as TFA salt in 7:3:2 BuOH:formic acid: water on TLC); reverse HPLC 1 ml/min using 0.04% solution of TFA sold in 40:60 water:acetonitrile yields 98% recovery. The active peptide ingredient of the pharmaceutical preparations according to the present invention may be used as a free peptide or in the form of a water soluble pharmaceutically acceptable salt, such as a sodium, potassium, ammonium or zinc salt. It will be understood that the pentapeptide may be administered with other active ingredients which independently impart an activity to the composition, such as, antibiotics, interferon, anesthetics, and the like.

The most preferred formulation according to the present invention is a solution for intramuscular injection containing about .001 to .01% by weight (.0001-.001mg/kg body weight, or 10-100µg active ingredient per 1ml solvent). The pharmaceutically acceptable vehicle for this injection form may be any pharmaceutically acceptable solvent such as 0.9% aqueous sodium chloride, distilled water, Novocaine solution, Ringer's solution, glucose solution, and the like. The peptide containing compositions according to the present invention may be administered in a compatible pharmaceutical suitable for parenteral administration (e.g., intravenous, subcutaneous, intramuscular). The preparations may be subjected to conventional pharmaceutical operations, such as sterilization, and may contain adjuvants, such as preservatives, stabilizers, wetting agents and the like.

The pharmaceutical preparations according to the present invention demonstrate a high effectiveness in the treatment of immunodepressed and immunodeficient states for the preventing and treatment of opportunistic infections in those states.

Also included within the scope of the present invention are the pharmaceutically acceptable salts of the peptide, such as sodium or potassium or strong organic bases, such as guanidine.

The peptide containing compositions according to the present invention have activity in the restoration and stimulation of the immune functions. Thus they are useful in the treatment of opportunistic infections of an immunodepressed subject in an immunopotentiating effective amount as described above.

The peptide compositions according to the present invention may also be used in veterinary practice as an immunomodulatory agent for prophylaxis and treatment of hypotrophy in farming animals, fur bearing animals and poultry.

Among the opportunistic infections which may be treated utilizing the compositions according to the present invention are: respiratory diseases, influenza, burns, wounds, other open sores, rashes (due to allergic reactions), sun exposure, local trauma (with an ointment), eczemas, psoriasis, and the like. Furthermore, the compositions according to the present invention may be utilized to assist healing in immunodepressed or immunodeficient states, such as for the healing of bone fractures, lesions, gingival diseases, gynecological infections, infralymphatic infections, and the like. The compositions may also be used to enhance the immunodeficient state to increase susceptibility to microbial antibiotics and to enhance the patient's responsive reaction to other types of therapies.

The composition of the present invention are useful for the treatment and prevention of states and diseases associated with homopoiesis reduction. In general, the compositions are useful for treatment and prevention of immunodepressive and immunodefficient states, primary or caused by acute or chronic diseases, including inflammatory infectitous and other diseases which may be accompanied by toxemia.

The compositions of the present invention are particularly useful for the treatment and prevention of anemias of all types such as those caused by blood loss, blood formation affection or blood destruction enhancement, including all types of iron deficient anemia, anemia associated with heme synthesis disorders, hemolytic and aplastic anemia, and primary and secondary anemia. Iron deficient anemia include chronic blood by menstruation, gastrointestinal bleeding, hemoptysis, hematuria, hemodialysis, malabsorption, infections, inborn iron deficiency, dietary iron deficiency and increased iron requirements during infancy, adolescence, pregnancy and lactation. Anemia also includes inherited hemolytic anemia such as those associated with abnormalities of the red cell membrane such as spherocytosis, stomatocytosis and elliptocytosis; anemia associated with erythrocytes enzyme deficiencies (glucose-6-phosphate dehydrogenase deficiency, pyruvate kinase deficiency, etc.) and also anemia associated with abnormal structure and synthesis of hemoglobin chains (such as thalassemia, sickle cell diseases, etc.). These hemolytic anemia also are treatable when associated with the symptom of antibody attack against erythrocytesor erythroid bone marrow cells, anemia associated with red cell membrane changes due to somatic mutations, paroxysmalnocturnal hemoglobinuria, anemia associated with mechanical red cell damage (artificial valves), anemia associated with chemical red cell damage (hemolytic poisons, lead, etc.) anemia associated with Vitamin E deficiency, anemia associated with thermal red cell damage and with parasites (including malaria), infections and hypersplenism. Anemia also include megaloblastic anemia such as those associated with vitamin B12 deficiency, addisonian/pernicious anemia), cancer of the stomach, intestinal diseases (intestinal tumors, sprue, terminal ileitis), gastrointestinal operations (gastroenterostomy, blind loop syndrome, resection of the ileum, etc.). Anemia conditioned by increase vitamin B12 expenditure and effects thereof on the bone marrow, including causes such as intestinal parasites (diphyllobothriasis), and liver diseases (hepatitis, cirrhosis, etc.), and hemoblastosis.

Other anemia may be caused by folic acid deficiency associated with intestinal diseases (intestinal surgery, intestinal malabsorption), long term drug administration (anti convulsants, etc.), dietary insufficiency, increased folic demand (infancy, adolescence, pregnancy, lactation), hemolytic anemia, psoriasis), excess loss of folate (hemodialysis, peritoneal dialysis), and defective folate synthesis (liver disease, alcoholism, antifolate drugs).

The invention is also useful for the treatment of T-cell immunodeficiencies such as sever combined immunodeficiency, Di George's syndrome, Wiskott-Aldrich syndrom, and chronic mucocutaneous candidiasis.

The peptides according to the present invention are also useful for the treatment and prevention of diseases which manifest bone marrow functional insufficiency (including aplastic anemia), such as that associated with radiation, chemical agents (benzene, trinitrotoluene, insecticides, etc.), antibodies against marrow cells, hereditary factors (Fanconi's anemia) or infections such as viral hepatitis.

In general, the peptides according to the present invention are useful for the treatment and prevention of all types of lympho- leuko- and cytopenias, including congenital conditions, and those caused by radiation or other cytotoxic factors and agents. Other conditions which may be treated include hemorrhagic diathesis, hemophilia, thrombocytopenic purpura, hemorrhagic vasculitis, DC, hereditary hemorrhagic telangiectasia.

Organ and tissue regeneration may also be stimulated by use of the peptides according to the present invention in states and diseases accompanied by restoration of tissue and integrity, including wound processes and toxic and infectious damage of cells. Also, the immune system and other defenses may be stimulated by the peptides according to the present invention during recovery to combat chronic diseases accompanied by toxemia (or tissue metabolism disturbances) or to assist in the adaptation to an new or extreme environmental condition. Immunodeficiencies associated with the use of drugs, including antibiotics and antineoplastic, or associated with the use of therapies, such as radiation and surgical intervention may also be alleviated by use of the peptides according to the present invention.

Tumor diseases and parasitic diseases, (such as helminthism) may also be treated as well as specific infections, such as tuberculosis, syphilis and gout. Acute and chronic poisoning may also be treated.

Agranulocytosis resulting from cytostatic factors (irradiation, cytotoxic drugs, drugs with cytotoxic side effects [such as chloramphenicol, chlorpromazine, etc.]) may also be treated. Immune agranulocytoses may also be treated and prevented, including those in systemic diseases, (SLE, hepatitis, etc.) or those caused by formation of anti-leukocytic antibodies under the influence of chemical compounds, such as drugs (sulfamides, barbiturates, aminopyrine, etc.).

States and diseases accompanied by neutropenia, may also be treated including infectious diseases (typhoid, tularemia, brucellosis), viral diseases (hepatitis, influenza, mumps, infectious mononucleosis), protozoa (malaria), chemical and physical agents (radiation, drugs), idiosyncratic drug reactions, hypersplenism (liver diseases, storage diseases), collagen-vascular diseases (SLE), sever folic acid or B12 deficiency, and neutropenia associated with extracorporalicirculation and pulmonary microcirculation disorders.

Pancytopenia may also be treated caused by hemopoiesis (megaloblastic anemia and myelodysplactic syndromes), hemodilution, hypersplenism and immune destruction.

Secondary immunodeficiencies such as viral infections (HIV, measles, cytomegalovirus, Epstein-Barr virus), splenectomy, burns, frost bites, wound healing processes, immunosuppressive drugs (antibiotics, corticosteroids, antimetabolites, etc.), radiation, prematurity, diabetes, protein-losing states, nephrotic syndrome, enteropathies, and aging.

Primary B-cell immunodeficiencies may be treated such as selective IgA deficiency, selective IgM deficiency, selective IgG sub class deficiency, X-linked agammaglobulinemia and variable hypogammaglobulinemia.

Finally, peptides according to the present invention may be used to treat surgical, gynecological and ENT purulent diseases.

### EXAMPLE 1

### EFFECT OF IMMUNE SYSTEM OF HEALTHY GUINEA PIGS

Forty male guinea pigs were used in the following test. Most of the animals were treated daily with a single dose (i.m.) of the pentapeptide (linear monomer Glx=Glu) of microgram/kg for five days. Control animals were treated with single daily doses of 0.5 ml (i.m.) of normal saline.

Tested parameters: clinical blood examination, non-specific resistance by lysosomal cationic test; "active" T-lymphocytes and total T-lymphocytes (E-RFC), B-lymphocytes (EAC-RFC) were measured in blood, thymus, lymph nodes, spleen and red bone marrow. Blood lymphocyte functional activity was evaluated by leukocyte migration inhibition (LMI) with ConA. Histological examinations of thymus, spleen, lymph nodes, bone marrow and adrenals were carried out. All of these indicia were measured on 10th and 20th days after onset of the treatment.

Principal findings: the peptide stimulates lymphoid cells proliferation and differentiation in thymus and bone marrow; on the 10th day the predominant T-lymphocyte stimulation is observed, on the 20th - both T- and B-lymphocytes. On the 10th day after onset of administration, the peptide causes increased level of mitotic activity in lymph nodes, spleen, and especially bone marrow.

### EXAMPLE 2

### EFFECT ON IMMUNE SYSTEM

Twenty-two male guinea pigs were used in the following test.

Guinea pigs were exposed to irradiation in a total dose 1Gy.target-skin distance - 70 cm; time of exposure - 2′48˝ Device: 180 kV; 15 mA; filter 0.5 Cu Treatment: i.m. single daily 1 microg. active ingredient per kg for 5 days

Treatment of controls: normal saline 0.5 ml i.m. single daily for 5 days.

Leukocyte and lymphocyte levels were measured in peripheral blood on the 7th, 14th, 21st, 36th and 44th day after irradiation.

Principal findings: the peptide stimulated proliferation of blood lymphoid cells resulted in restoration of leukocyte and lymphocyte levels. In controls there were no immune cells restoration during all period of observation.

In a second test 40 male guinea pigs were used, and the same regimen was followed.

There were two controls - irradiated and non-irradiated. Parameters were evaluated on the 8th and 21st days after irradiation. Tested parameters: clinical blood examination, non-specific resistance by lysosomal cationic test; "active" T-lymphocytes and total T-lymphocytes (E-RFC), B-lymphocytes (EAC-RFC) were measured in blood, thymus, lymph nodes, spleen and red bone marrow. Blood lymphocyte functional activity was evaluated by leukocyte migration inhibition (LMI) with ConA. Histological examinations of thymus, spleen, lymph nodes, bone marrow and adrenals were carried out.

Principal findings: the peptide use in irradiated animals accelerates T-lymphocyte maturation and their migration to peripheral immune organs in early terms of observation. In the later stage of the study effects were more pronounced in the enhancement of proliferation and differentiation in both central and peripheral organs of the immune system. Administration restored peripheral blood lymphocytes and neutrophil functional activity.

### EXAMPLE 3

### EFFECTS IN THYMECROMIZED GUINEA PIGS

Model: Thymectomy (removal of thymus): 30 mongrel male guinea pigs

Treatment: i.m. single daily 1 microg active ingredient per kg for 10 days

Treatment of controls: normal saline 0.5 ml i.m. single daily for 10 days (there were two controls - thymectomized and sham-operated).

Parameters were determined on the 15th day after onset of the treatment.

Tested parameters: clinical blood examination, "active" T-lymphocytes and total T-lymphocytes (E-RFC), B-lymphocytes (EAC-RFC) were measured in blood, thymus, lymph nodes, spleen and red bone marrow.

Principal findings: the peptide use in thymectomized animals does not stimulate lymphoid cells differentiation, but, on the contrary, does suppress it to some degree.

### EXAMPLE 4

### EFFECT ON SUPERFICIAL RECEPTORS

### EXPRESSION ON T- AND B-LYMPHOCYTES

Model: A. This work was designed to study the restoration of superficial receptors on lymphocytes after proteolytic digestion or after severe secondary immunodeficiency. Thymocytes obtained from guinea-pig were trypsinized and then their rosette-forming capacity with rabbit erythrocytes (E-RFC) was evaluated. The cells were incubated with the peptide in concentrations 1, 10 and 100 microg/ml. There were two controls - intact thymocytes (not trypsinized) and trypsinized thymocytes not incubated with the peptide.

Principal findings: The peptide was the most active in concentration 10 microg/ml - its biological activity made up 78.9% (percentage of rosette-forming capacity restoration).

B. B-lymphocytes were obtained from patients with streptococcal and staphylococcal skin diseases showed highly pronounced secondary immunodeficiency. The number of cells carrying Ig-receptors before and after incubation with the peptide has been measured (by means of FITC-labelled sera against human Ig).

Principal findings: the peptide in concentration 1 microg/ml causes significant increase of cells carrying Ig-receptors of different types.

### EXAMPLE 5

### ERYTHROPOIETIC EFFECTS

This test was designed to study posthemorrhagic anemia (acute blood loss caused by taking blood from retroorbital sinus), and hemolytic anemia induced by phenylhydrazine hydrochloride (120 mg active ingredients/kg 30 Balb/c-mice and 30 CBA-mice).

The peptide was injected intraperitoneally in doses 100 and 150 microg per kg, 3 hours and 1 day after intervention modelling anemia, for 5 days.

Tested parameters: RBC, leukocytes, reticulocytes, Hb, Hct

Principal findings: 1. in posthemorrhagic anemia the most pronounced alterations of tested parameters were observed on 4th-5th day after the invasion: erythrocytes dropped to 4.2 mln/ml vs. 6.2 mln/ml in control, reticulocytes rose 3 times, leukocytes were also increased. After hg administration, on the 6th day, RBC count rose up to 7.1 mln/ml; Hb level and plasma/formed elements ration restoration were more rapid. The peptide was the most effective in dose 150 microg/kg.

2. In hemolytic phenylhydrazine-induced anemia the most pronounced hemodepression has arisen on the 7th day. RBC dropped to 3.8 mln/ml, Hb was diminished by 15%, reticulocytes have grown up to 15%. On the 3rd day of administration erythrocytes have increased up to 7.2 mln/ml and remained on this level in later terms. Thus, the peptide has erythropoietic effect in anemias of different genesis.

### EXAMPLE 6

### INFLUENCE ON COLONY-FORMING ACTIVITY

This test is designed to study macrophage precursors. Cultured cells used were guinea-pig myelokaryocytes. The peptide was added to cell culture in concentrations 1.0, 0.001, 0.00001 and 0.0000001 microg/ml.

The peptide stimulates macrophage precursors colony-forming activity in concentration starting from 0.0000001 microg/ml.

### EXAMPLE 7

### HEMOSTIMULATING EFFECT

The test was designed to study hemodepression induced by 5-fluorouracil injected i.p. in a dose 175 mg per kg (172 male CBA-mice). Treatment: the peptide was administered i.p. starting from 4th day after 5-FU injection in doses 0.00001, 0.001, 0.01, 0.1, 1.0 mg/kg for 5 days.

Treatment of controls: normal saline i.p. for 5 days.

Tested parameters: peripheral blood count and bone marrow differential count.

Principal findings: the peptide use promotes active restoration of hemopoiesis. This resulted in normalization of leukocytes and all CBC parameters. In bone marrow the peptide causes restoration of cellularity normalization of all lines of hemopoiesis. The peptide was active starting from 0.001 mg/kg.

## Claims

1. A pharmaceutical preparation comprising a therapeutically effective amount to lower a hyperactive immune state or to restore normal immunological indices of a subject of the pentapeptide L-Thr-L-Ala-L-Glx-L-Glx-L-Lys and/or its pharmaceutically acceptable salts in a pharmaceutically acceptable vehicle; wherein Glx is Gln or Glu.

2. The preparation of claim 1 in the form of an injection solution comprising 0.001 to 0.1% by weight of the active ingredient.

3. The preparation of claim 1 in the form of tablets, suppositories, capsules, eye films, inhalant, mucosal spray, toothpaste, ointments, and/or water soluble based creams.

4. The use of a peptide for making a pharmaceutical preparation for the treatment and/or prevention of opportunistic infections in an immunodepressed or immunodeficient subject, wherein the peptide is selected from the group consisting of L-Thr-L-Ala-L-Glx-L-Glx-L-Lys and a pharmaceutically acceptable salt thereof, wherein Glx is Glu or Gln.

5. The use of a peptide for making a pharmaceutical preparation for the treatment of an immunodeficient, immunodepressed or hyperactive immune state in a subject, wherein the peptide is a pentapeptide selected from the group consisting of L-Thr-L-Ala-L-Glx-L-Glx-L-Lys and a pharmaceutically acceptable salt thereof, wherein Glx is Glu or Gln.

6. The use of claim 5 wherein said state comprises acquired hemopoiesis reduction.

7. The use of a peptide for making a pharmaceutical preparation for the treatment or prevention of anaemia, wherein the peptide is a pentapeptide selected from the group consisting of L-Thr-L-Ala-L-Glx-L-Glx-L-Lys and a pharmaceutically acceptable salt thereof, wherein Glx is Glu or Gln.

8. The use of a peptide for making a pharmaceutical preparation for the treatment of conditions of T-cell immunodeficiency in a subject, wherein the peptide is a pentapeptide selected from the group consisting of L-Thr-L-Ala-L-Glx-L-Glx-L-Lys and a pharmaceutically acceptable salt thereof, wherein Glx is Glu or Gln.

9. The use of a peptide for making a pharmaceutical preparation wherein the peptide is a pentapeptide selected from the group consisting of L-Thr-L-Ala-L-Glx-L-Glx-L-Lys and a pharmaceutically acceptable salt thereof, wherein Glx is Glu or Gln.

10. The use of a peptide for making a pharmaceutical preparation for treatment of the condition of lymphocytopenia wherein the peptide is a pentapeptide selected from the group consisting of L-Thr-Ala-L-Glx-L-Glx-L-Lys and a pharmaceutically acceptable salt thereof, wherein Glx is Gln or Glu.

11. The pentapeptide L-Thr-L-Ala-L-Glx-L-Glx-L-Lys and/or its pharmaceutically acceptable salts wherein Glx is Gln or Glu.

12. The pentapeptide of claim 11 wherein the compound is L-Thr-L-Ala-L-Glu-L-Glu-L-Lys or a pharmaceutically acceptable salt thereof.

## Patentansprüche

1. Pharmazeutische Zubereitung, die eine zur Senkung eines hyperaktiven Immunzustandes oder zur Wiederherstellung normaler immunologischer Werte eines Patienten therapeutisch wirksame Menge des Pentapeptids L-Thr-L-Ala-L-Glx-L-Glx-L-Lys und/oder seiner pharmazeutisch verträglichen Salze in einem pharmazeutisch verträglichen Träger enthält, wobei Glx für Gln oder Glu steht.

2. Zubereitung nach Anspruch 1 in Form einer Injektionslösung, die 0,001 bis 0,1 Gew-% des Wirkstoffs enthält.

3. Zubereitung nach Anspruch 1 in Form von Tabletten, Zäpfchen, Kapseln, Augenfolien, Inhalationsmittel, Schleimhautspray, Zahnpasta, Salben und/oder Cremes auf wasserlöslicher Basis.

4. Verwendung eines Peptids zur Herstellung einer pharmazeutischen Zubereitung für die Behandlung und/oder Vorbeugung von opportunistischen Infektionen bei einem immundefizienten oder immungeschwächten Patienten, wobei das Peptid ausgewählt ist aus der Gruppe bestehend aus L-Thr-L-Ala-L-Glx-L-Glx-L-Lys und einem pharmazeutisch verträglichen Salz desselben, wobei Glx für Glu oder Gln steht.

5. Verwendung eines Peptids zur Herstellung einer pharmazeutischen Zusammensetzung für die Behandlung eines immundefizienten, immungeschwächten oder hyperaktiven Immunzustandes bei einem Patienten, wobei das Peptid ein Pentapeptid ist, das ausgewählt ist aus der Gruppe bestehend aus L-Thr-L-Ala-L-Glx-L-Glx-L-Lys und einem pharmazeutisch verträglichen Salz desselben, wobei Glx für Glu oder Gln steht.

6. Verwendung nach Anspruch 5,
wobei der Zustand eine erworbene Hämatopoesereduzierung ist.

7. Verwendung eines Peptids zur Herstellung einer pharmazeutischen Zubereitung für die Behandlung oder Vorbeugung von Anämie, wobei das Peptid ein Pentapeptid ist, das ausgewählt ist aus der Gruppe bestehend aus L-Thr-L-Ala-L-Glx-L-Glx-L-Lys und einem pharmazeutisch verträglichen Salz desselben, wobei Glx für Glu oder Gln steht.

8. Verwendung eines Peptids zur Herstellung einer pharmazeutischen Zusammensetzung für die Behandlung der Bedingungen von T-Zellen-Immundefizienz in einem Patienten, wobei das Peptid ein Pentapeptid ist, das ausgewählt ist aus der Gruppe bestehend aus L-Thr-L-Ala-L-Glx-L-Glx-L-Lys und einem pharmazeutisch verträglichen Salz desselben, wobei Glx für Glu oder Gln steht.

9. Verwendung eines Peptids zur Herstellung einer pharmazeutisch verträglichen Zusammensetzung, wobei das Peptid ein Pentapeptid ist, das ausgewählt ist aus der Gruppe bestehend aus L-Thr-L-Ala-L-Glx-L-Glx-L-Lys und einem pharmazeutisch verträglichen Salz desselben, wobei Glx für Glu oder Gln steht.

10. Verwendung eines Peptids zur Herstellung einer pharmazeutischen Zusammensetzung für die Behandlung des Zustandes von Lymphozytopenie, wobei das Peptid ein Pentapeptid ist, das ausgewählt ist aus der Gruppe bestehend aus L-Thr-L-Ala-L-Glx-L-Glx-L-Lys und einem pharmazeutisch verträglichen Salz desselben, wobei Glx für Glu oder Gln steht.

11. Das Pentapeptid L-Thr-L-Ala-L-Glx-L-Glx-L-Lys und/oder seine pharmazeutisch verträglichen Salze, wobei Glx für Gln oder Glu steht.

12. Das Pentapeptid nach Anspruch 11,
wobei die Verbindung L-Thr-L-Ala-L-Glu-L-Glu-L-Lys oder ein pharmazeutisch verträgliches Salz desselben ist.

## Revendications

1. Une préparation pharmaceutique comprenant une quantité, thérapeutiquement efficace pour abaisser un état immunitaire hyperactif ou pour restaurer des indices immunologiques normaux d'un sujet, d'un pentapeptide L-Thr-L-Ala-L-Glx-L-Glx-L-Lys et/ou un de ses sels pharmaceutiquement acceptables dans un véhicule pharmaceutiquement acceptable ; où Glx est Gln ou Glu.

2. La préparation selon la revendication 1 sous la forme d'une solution injectable comprenant 0,001 à 0,1 % en poids d'ingrédient actif.

3. La préparation selon la revendication 1 sous la forme de comprimés, suppositoires, capsules, films oculaires, inhalations, pulvérisations mucosales, pâtes dentifrices, pommades et/ou crèmes hydrosolubles.

4. L'utilisation d'un peptide pour préparer une préparation pharmaceutique pour le traitement et/ou la prévention d'infections opportunistes chez un sujet immunodéprimé ou immunodéficient, dans laquelle le peptide est choisi dans le groupe consistant en L-Thr-L-Ala-L-Glx-L-Glx-L-Lys et un de ses sels pharmaceutiquement acceptables, où Glx représente Glu ou Gln.

5. L'utilisation d'un peptide pour préparer une préparation pharmaceutique pour le traitement d'un état immun immunodéficient, immunodéprimé ou hyperactif chez un sujet, dans laquelle le peptide est un peptide choisi dans le groupe consistant en L-Thr-L-Ala-L-Glx-L-Glx-L-Lys et un de ses sels pharmaceutiquement acceptable où Glx représente Glu ou Gln.

6. L'utilisation selon la revendication 5 dans lequel ledit état comprend la réduction de l'hématopoièse acquise.

7. L'utilisation d'un peptide pour préparer une préparation pharmaceutique pour le traitement ou la prévention de l'anémie, dans laquelle le peptide est un pentapeptide choisi dans le groupe consistant en L-Thr-L-Ala-L-Glx-L-Glx-L-Lys et un de ses sels pharmaceutiquement acceptables, où Glx représente Glu ou Gln.

8. L'utilisation d'un peptide pour préparer une préparation pharmaceutique pour le traitement d'états d'immunodéficience des cellules T chez un sujet, dans laquelle le peptide est un pentapeptide choisi dans le groupe consistant en L-Thr-L-Ala-L-Glx-L-Glx-L-Lys et un de ses sels pharmaceutiquement acceptables, où Glx représente Glu ou Gln.

9. L'utilisation d'un peptide pour préparer une préparation pharmaceutique dans laquelle le peptide est un pentapeptide choisi dans le groupe consistant en L-Thr-L-Ala-L-Glx-L-Glx-L-Lys et un de ses sels pharmaceutiquement acceptables où Glx représente Glu ou Gln.

10. L'utilisation d'un peptide pour préparer une préparation pharmaceutique pour le traitement d'états de lymphocytopénie dans laquelle le peptide est un pentapeptide choisi dans le groupe consistant en L-Thr-L-Ala-L-Glx-L-Glx-L-Lys et un de ses sels pharmaceutiquement acceptables, où Glx représente Glu ou Gln.

11. Le pentapeptide L-Thr-L-Ala-L-Glx-L-Glx-L-Lys et/ou ses sels pharmaceutiquement acceptables où Glx est Gln ou Glu.

12. Le pentapeptide selon la revendication 11, dans lequel le composé est le en L-Thr-L-Ala-L-Glx-L-Glx-L-Lys et un de ses sels pharmaceutiquement acceptables.
